(19) Europäisches Patentamt
European Patent Office
Office européen des brevets

(11) Numéro de publication: **0 371 837 A2**

# (12) DEMANDE DE BREVET EUROPEEN

(21) Numéro de dépôt: **89403023.8**

(22) Date de dépôt: **02.11.89**

(51) Int. Cl.[5]: **C07H 13/12, A61K 31/70, C07D 317/24, A61K 31/335, C07C 333/16, C07C 333/18, C07C 333/20, A61K 31/27**

(30) Priorité: **02.11.88 FR 8814276**

(43) Date de publication de la demande: **06.06.90 Bulletin 90/23**

(84) Etats contractants désignés: **AT BE CH DE ES FR GB GR IT LI LU NL SE**

(71) Demandeur: **INSTITUT MERIEUX INTERNATIONAL**
**17 rue Bourgelat**
**F-69002 Lyon(FR)**

(72) Inventeur: **Postel, Denis Ghislain**
**43 rue Juliette Nevouet**
**F-60000 Goincourt(FR)**
Inventeur: **Ronco, Gino Lino**
**11 Bd Pont Noyelles**
**F-80000 Amiens(FR)**
Inventeur: **Villa, Pierre Joseph**
**43 rue Maréchal de Castries**
**F-80000 Amiens(FR)**
Inventeur: **Ville, Guy André**
**15 rue Charles Friedel**
**F-75020 Paris(FR)**
Inventeur: **Plan, Robert**
**110 rue Sully**
**F-69006 Lyon(FR)**

(74) Mandataire: **Bernasconi, Jean et al**
**CABINET LEMOINE ET BERNASCONI 13,**
**Boulevard des Batignolles**
**F-75008 Paris(FR)**

## (54) Procédé de synthèse spécifique de nouveaux esters d'acides dithiocarbamiques par substitution de sites hydroxylés sur des molécules mono- ou polyhydroxylées produits obtenus for ce procédé et leurs applications.

(57) Procédé de synthèse spécifique d'esters d'acides dithiocarbamiques par substitution de sites hydroxylés sur des molécules mono- ou polyhydroxylées, produits obtenus par ce procédé et leurs applications.

Procédé de préparation d'esters d'acides dithiocarbamiques de formule

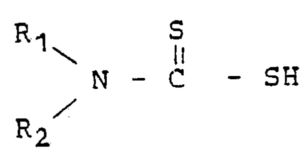

dans laquelle $R_1$ et $R_2$ sont des radicaux alkyle saturés ou non, substitués ou non avec des groupements carbonés ou des hétéroatomes ou encore formant avec l'atome d'azote un cycle saturé ou non comportant ou non des hétéroatomes à partir d'alcools ou de polyols, caractérisé en ce que l'on transforme, dans une étape préliminaire, au moins un groupement hydroxylé de la molécule mono- ou polydroxylée en un groupement partant (Y) tel qu'un iodure, tosylate, brosylate, triflate, ou imidazole sulfonate et en ce que l'on déplace le goupement partant (Y) par l'anion

EP 0 371 837 A2

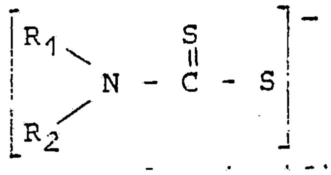

$R_1$ et $R_2$ ayant la signification précitée, introduit sous forme de sel, de préférence de lithium, dans le milieu approprié. Les nouveaux dérivés obtenus sont utilisables comme médicaments, notamment immunomodulateurs.

**Procédé de synthèse spécifique de nouveaux esters d'acides dithiocarbamiques par substitution de sites hydroxylés sur des molécules mono- ou polyhydroxylées, produits obtenus par ce procédé et leurs applications.**

La présente invention a trait à un procédé de préparation spécifique de nouveaux esters d'acides dithiocarbamiques à partir de molécules mono- et polyhydroxylées.

Elle concerne également les produits obtenus par ce procédé ainsi que leurs applications, notamment à titre de médicaments.

On sait que les acides N,N-dialkyl dithiocarbamiques et leurs sels sont utilisés comme agents chélatants et complexants, notamment vis-à-vis des métaux de transition. On sait que ces composés présentent également d'intéressantes propriétés biologiques et qu'ils sont utilisés comme radioprotecteurs, comme agents antiviraux et antitumoraux.

Il est connu que la synthèse d'esters d'acides dithiocarbamiques ne peut être réalisée par action directe des acides dithiocarbamiques sur le ou les groupements OH d'alcools ou polyols.

L'un des buts de la présente invention est de proposer un procédé de préparation spécifique d'esters d'acides dithiocarbamiques à partir de molécules mono- ou polyhydroxylées avec des rendements élevés même sur les sites hydroxylés secondaires autres qu'anomériques, et qui soit d'une mise en oeuvre aisée.

Un autre but de l'invention est de fournir de nouveaux esters dithiocarbamiques qui, de par leurs propriétés biologiques, trouvent une application particulièrement intéressante comme médicaments immunomodulateurs et antiviraux.

Le procédé conforme à l'invention est caractérisé en ce que l'on transforme, dans une étape préliminaire, au moins un groupement hydroxyle de molécules mono- ou polyhydroxylées en un groupement partant (Y), puis en ce que l'on déplace le groupement partant (Y) par l'anion

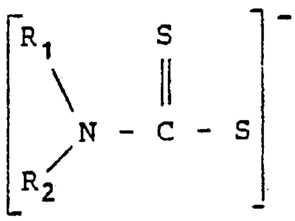

$R_1$ et $R_2$ représentant des radicaux alkyle saturés ou non, substitués ou non avec des groupements carbonés ou des hétéroatomes ou encore formant avec l'atome d'azote un cycle saturé ou non comportant ou non des hétéroatomes, introduit sous forme de sel dans le milieu approprié.

Le groupement partant (Y) est, de préférence, constitué par un halogénure tel qu'un iodure, ou encore par un sulfonate tel que brosylate tosylate, triflate, imidazole sulfonate.

Pour préparer les produits intermédiaires notés MY qui résultent de la transformation des groupements hydroxyle en groupements partant (Y), on peut mettre en oeuvre des procédés connus.

On peut utiliser par exemple :

- pour les dérivés iodés, le procédé décrit dans la demande de brevet français n° 83 05230 déposée le 20 avril 1988 au nom de l'Université de Picardie,
- pour les dérivés chlorés et bromés, le procédé décrit Par HANESSIAN dans Carbohydrate Research 24, p. 45 (1972),
- pour les esters sulfoniques, le procédé décrit dans la demande de brevet français n° 88 05231 déposée le 20 avril 1988 au nom de l'Université de Picardie.

Dans le cas des molécules polyhydroxylées notées $[Su(OH)_n]$, on pourra obtenir la dithiocarbamatation sur un ou plusieurs des sites hydroxylés. Si l'on désire obtenir la dithiocarbamatation sur un site OH seulement, il convient de protéger les autres groupements OH.

Appliqué à la préparation de monoesters d'acides dithiocarbamiques à partir de polyols $[Su(OH)_n]$, le procédé consiste successivement :

- à protéger sélectivement (n-1) groupements OH à l'aide d'une cétone ou d'un aldehyde pour donner $[Su-(O-A)_{n-1}-OH]$,
- à transformer le groupement OH en un groupement partant (Y) pour donner $[Su\ (O-A)_{n-1}-\ Y)]$, noté [Su P-Y],
- à estérifier par action sur [Su P-Y] d'un sel de l'acide dithiocarbamique de formule

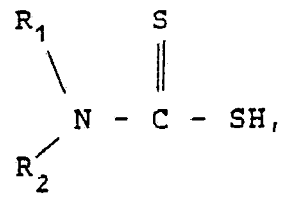

pour former l'ester

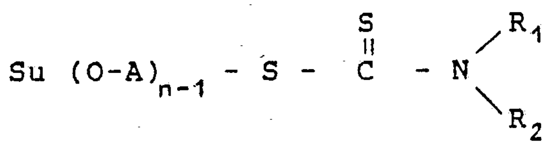

- et à libérer les sites hydroxylés préalablement protégés sous forme de dioxolanne.

La régénération des sites hydroxylés pourra être partielle ou totale afin d'obtenir des monoesters dithiocarbamiques aux propriétés physico-chimiques différentes, notamment par leurs hydrosolubilité et liposolubilité.

Parmi les molécules polyhydroxylées qui conviennent particulièrement bien à la réalisation de l'invention, on peut citer le glycérol, le galactose, le glucose, le mannose, le fructose, des itols et, d'une façon plus générale, des mono-, di- ou polysaccharides.

La préparation des esters dithiocarbamiques, à partir de l'halogénure ou du sulfonate [$Su(OA)_{n-1}$ - Y], noté [Su P-Y], comprend les étapes suivantes :

- de mise en contact de l'halogénure ou du sulfonate [Su P-Y] et d'un sel de l'acide dithiocarbamique en présence d'un solvant aprotique polaire, le sel de l'acide étant utilisé en excès par rapport à la quantité stoechiométrique,
- d'élimination du solvant et de reprise du résidu par un solvant organique,
- d'élimination du sel de l'acide dithiocarbamique résiduel,
- d'évaporation de la phase organique contenant l'ester dithiocarbamique,
- de purification dudit ester,
- et, éventuellement, de déprotection partielle ou totale des sites OH bloqués sous forme de dioxolanne.

Le sel de l'acide dithiocarbamique que l'on fait réagir avec l'intermédiaire [Su P-Y] est avantageusement constitué par un sel de métal alcalin tel qu'un sel de lithium, de sodium ou de potassium.

Ce sel de l'acide dithiocarbamique est ajouté au solvant aprotique polaire dans un rapport molaire généralement compris entre 1,1 et 2, et de préférence voisin de 1,5 par rapport à l'intermédiaire [$Su(O-A)_{n-1}$ -Y].

On peut utiliser comme solvant aprotique polaire : l'acétone, l'hexaméthyl phosphorotriamide (HMPA), le diméthylformamide (DMF), le diméthoxyéthane (DME), le diméthylsulfoxyde (DMSO) ou encore un mélange de ces solvants. Il est également possible d'associer l'un de ces solvants ou le mélange de ces solvants à un autre solvant organique qui peut être un solvant aromatique tel que le toluène, le xylène, le benzène ou un hydrocarbure saturé ou encore un mélange d'hydrocarbures saturés.

Après réaction du sel de l'acide dithiocarbamique avec l'intermédiaire [Su P-Y] et élimination du solvant aprotique, le résidu est repris par un solvant organique tel que l'éther éthylique qui solubilise l'ester, l'acide dithiocarbamique qui n'a pas réagi étant éliminé par filtration.

Après évaporation de la phase organique qui contient l'ester, on purifie celui-ci par recristallisation ou par chromatographie liquide sur gel de silice.

Dans le cas où les esters dithiocarbamiques sont obtenus en faisant réagir le sel de l'acide dithiocarbamique avec un ester sulfonique (le groupe partant (Y) est un sulfonate), on procède, après l'achèvement de la réaction, à une filtration afin d'éliminer le sulfonate salin formé.

Dans le cas où l'on part de molécules polyhydroxylées dont les sites OH ne devant pas être estérifiés ont été bloqués, on effectue la déprotection partielle ou totale de ces sites OH bloqués soit en acido-catalyse hétérogène par passage d'une solution de l'ester dithiocarbamique dans un mélange de solvants alcanol/eau, alcanol pur ou mélange d'alcanols sur une colonne remplie de résine acide maintenue à une température contrôlée, soit en acido- catalyse homogène dans les mêmes solvants en présence d'un acide minéral fort à la concentration 0,05 à 1N, sous agitation à une même température contrôlée et pendant la

durée appropriée. On obtient, selon les conditions opératoires, une déprotection partielle ou totale des fonctions hydroxyle.

Les esters dithiocarbamiques obtenus par le procédé conforme à l'invention et qui sont des esters nouveaux, présentent des propriétés similaires à celles du diéthyldithiocarbamate de sodium (DEDTC-Na) disponible dans le commerce, mais avec une action thérapeutique retardée, une moindre toxicité et une administration facilitée par la modulation des propriétés chimiques et physiques par choix de la molécule mono- ou polyhydroxylée de départ.

Les esters dithiocarbamiques conformes à l'invention trouvent une application intéressante dans la préparation de produits immunomodulateurs. De tels médicaments peuvent être avantageusement utilisés pour le traitement et la prévention d'affections liées à des dérèglements immunitaires chez l'enfant ou le nourrisson, et notamment la mucoviscidose, les infections respiratoires récidivantes, la polyarthrite juvénile. Ces médicaments peuvent être également utiles dans le cas de maladies auto-immunes des adultes et notamment la polyarthrite rhumatoïde, le lupus érythémateux disséminé, des infections bactériennes au long court ou récurrentes, des complications septiques de la bronchite chronique, la bird shot rétinopathie et des déficiences en lymphocytes.

Ces esters dithiocarbamiques trouvent également une application intéressante dans la préparation d'un médicament actif dans la prévention et le traitement des maladies virales, notamment celles dues aux virus HIV1 ou 2.

Les esters de l'acide dithiocarbamique conformes a l'invention sont administrés à des doses généralement comprises entre 1 et 500 mg/kg de poids corporel.

L'administration de ces médicaments peut être, par exemple, effectuée par voie orale, soit sous forme d'une solution, soit sous forme solide, par exemple sous forme de gélules gastroprotégées.

Il va être donné, ci-dessous, quelques exemples de mise en oeuvre de l'invention, à titre illustratif, mais non limitatif. Pour ces exemples l'intermédiaire de type [Su(OA)$_{n-1}$ - OH] est soit un produit commercial, soit un produit préparé par protection de sites hydroxylés sous forme de dioxolanne à partir de molécules polyhydroxylées [Su(OH)$_n$] selon les méthodes classiques de la littérature.

L'intermédiaire de type [Su P-Y] est soit un tosylate, soit un mésylate, soit un triflate, soit un iodure.

Le tosylate est préparé selon le brevet FR 88 05231 :

On additionne dans un ballon, contenant 200 ml d'acétone anhydre, maintenu à 0 °C, 0,1 mol de molécule hydroxylée (Su (OA)$_{n-1}$ -OH] , 1,11 mol de triéthylamine, et 0,11 mol de chlorure de paratoluène sulfonyle, sous agitation. On laisse revenir le mélange à température ambiante en maintenant l'agitation pendant 10 heures. On élimine les cristaux de chlorhydrate de triéthylamine par filtration. Le filtrat est évaporé sous pression réduite, le résidu est recristallisé 3 fois dans le mélange hexane/éther éthylique, 50/50 (V/V). Les rendements en tosylate pur sont compris entre 60 et 90 %.

Le mésylate est préparé à partir du chlorure de méthane sulfonyle en remplaçant dans le protocole précédent l'acétone par l'éther éthylique/hexane, 50/50 (V/V) et en ramenant le temps de réaction à 1 heure au lieu de 10 heures. Lorsque le point de fusion du mésylate est bas, la purification est réalisée sur gel de silice. Les rendements en mésylate pur sont compris entre 75 et 95 %.

Le triflate est préparé selon la méthode de FLECHNER (carbohydrate Research 77, (1979), 262-266) par addition de [Su (OA)$_{n-1}$-OH] à une solution refroidie d'anhydride triflique dans le dichlorométhane en présence de pyridine. Après 90 mn de réaction à -10 °C, on neutralise avec $NaHCO_3$, on lave la phase organique avec une solution aqueuse d'HCl à 3 %, on purifie par chromatographie sur silice ou par recristallisation.

L'iodure est préparé selon le brevet FR 88 05230 :

On additionne dans un ballon muni d'un réfrigérant, contenant 500 ml de xylène, 0,1 mol de molécule hydroxylée [Su (OA)$_{n-1}$ - OH] , 0,105 mol de triphénylphosphine et 0,2 mol d'imidazole. A ce mélange, porté à 80 °C sous agitation, est ajouté 0,1 mol d'iode, puis on porte l'ensemble à reflux pendant 3 heures. Le mélange réactionnel est ensuite transvasé dans un erlen de 2 litres, où l'on ajoute 500 ml de solution aqueuse saturée de $HNaCO_3$ . Après 10 mn d'agitation, on ajoute de l'iode jusqu 'à coloration de la phase organique, puis la quantité nécessaire de thiosulfate de sodium en solution aqueuse pour observer une décoloration. La phase organique est décantée, séchée sur $Na_2SO_4$, filtrée et évaporée sous pression réduite. Le résidu est purifié soit par deux recristallisations successives dans le mélange hexane/éther éthylique, 90/10, (V/V), soit par chromatographie sur gel de silice. Les rendements sont compris entre 60 et 90 %.

Exemple 1 :

Synthèse du désoxy-1 N,N-diéthyl dithiocarbamate O-isopropylidène-2,3 glycérol à partir de sulfonate de O-isopropylidène-2,3 glycérol et de désoxy-1 iodo-1-Oisopropylidène-2,3 glycérol.

25 g ($8,2.10^{-2}$ mol) de tosylate de O-isopropylidène-2,3 glycérol (tosylate de solkétal) et 27,75 g ($12,3.10^{-2}$mol) de N,N-diéthyl dithiocarbamate de sodium trihydraté (DEDTC-Na) sont placés dans un tricol de 1 litre contenant 500 ml d'acétone. Sous agitation, le mélange est porté à reflux ; le contrôle de l'avancement de la réaction s'effectue par HPLC.

8 heures après le début du reflux, on observe un taux de conversion du tosylate de solkétal de 96 %.

Après retour à la température ambiante, on filtre sur un fritté n° 4, le précipité étant du tosylate de sodium, le filtrat contenant le dérivé diéthyldithiocarbamique.

L'acétone est éliminée sous pression réduite.

Le résidu est repris par de l'éther éthylique qui solubilise sélectivement l'ester, le DEDTC-Na étant éliminé par filtration.

L'éther éthylique est éliminé sous pression réduite, le brut (24,09 g) qui se présente sous forme d'une huile, est purifié par HPLC.

Le brut est introduit dans l'éluant qui est le mélange hexane industriel/acétone 93/7 (V/V) sur une colonne de gel de silice. On obtient 17,85 g (94,2 %) de N,N-diéthyl dithiocarbamate de O-isopropylidène-2,3 glycérol (DEDTC de solkétal) pur d'après les analyses HPLC, RMN $^{1}H$ et $^{13}C$.

La synthèse a été aussi effectuée selon le protocole décrit précédemment, à partir du mésylate de O-isopropylidène-2,3 glycérol (mésylate de solkétal). Après 24 heures de réaction, le rendement n'est que de 40 % en diéthyldithiocarbamate de solkétal. En remplaçant le solvant acétone pur par un mélange acétone/HMPA 80/20 (V/V), on obtient, après 24 heures, un taux de conversion du mésylate de solkétal de 90 %.

La synthèse a été effectuée également selon le protocole précédemment décrit, à partir du désoxy-1 iodo-1 O-isopropylidène-2,3 glycérol (iodo-solkétal). Après 12 h dans l'acétone à reflux, la conversion de l'iodo solkétal est totale. On obtient après purification 96 % de N,N-diéthyldithiocarbamate de solkétal.

On donne dans les tableaux I et II respectivement les caractéristiques des spectres RMN$^{1}$H et RMN$^{13}$C du DEDTC de solkétal obtenu.

A partir du tosylate, du mésylate et de l'iodo solkétal, on a préparé, selon le même protocole, le désoxy-1 N,N diméthyl dithiocarbamate O-isopropylidène 2,3 glycérol (DMDTC de solkétal) avec des rendements supérieurs à 90 % (pureté contrôlée par HPLC et RMN).

Exemple 2 :

Synthèse du désoxy-6 N,N-diéthyldithiocarbamate di-O-isopropylidène-1,2: 3,4-α-D galactopyrannose à partir du désoxy-6 iodo-6 di-O-isopropylidène 1,2:3,4-α-D galactopyrannose.

25 g (6,7.10 mol) de désoxy-6 iodo-6 di-O-isopropylidène 1,2:3, 4-α-D galactopyrannose (iodo diacétone galactose) et 22,78 g ($10,1.10^{-2}$ mol) de DEDTC-Na sont placés dans un tricol de 1 litre contenant 500 ml d'acétone. Sous agitation, le mélange est porté à reflux ; le contrôle d'avancement de la réaction s'effectue par HPLC. 24 heures après le début du reflux, on observe une disparition complète du iodo diacétone galactose. On évapore l'acétone sous pression réduite. On reprend le résidu par de l'éther éthylique ; on élimine par filtration le DEDTC-Na n'ayant pas réagi. Le filtrat est évaporé sous pression réduite. On obtient 26 g de brut. On opère la purification par une double recristallisation dans l'hexane industriel.

On obtient 24,1 g (92 %) de N,N-diéthyldithiocarbamate de diacétone galactose pur d'après les analyses HPLC, RMN $^{1}H$, RMN $^{13}C$.

F = 84° C ; $[\alpha]_D^{20}$ = -13,6° ($CHCl_3$).

On donne dans les tableaux III et IV respectivement les caractéristiques des spectres RMN $^{1}H$ et RMN $^{13}C$ du DEDTC diacétone galactose obtenu.

Exemple 3 :

Synthèse du désoxy-6 N,N-diméthyldithiocarbamate di-O-isopropylidène-1,2:3,4-α-D galactopyrannose à partir du désoxy-6 iodo-6 di-O-isopropylidène 1,2:3,4-α-D galactopyrannose.

2,9 g ($7,84.10^{-3}$ mol) de désoxy-6 iodo-6 di-O-isopropylidène 1,2:3,4-α-D galactopyrannose et 2,1 g (11,7.10 mol) de diméthyldithiocarbamate de sodium dihydraté sont placés dans un ballon de 100 ml contenant 58 ml d'acétone. Sous agitation, le mélange est porté à reflux.

Après 24 heures, on observe une disparition complète du substrat, on arrête la réaction, on extrait le mélange, selon le protocole précédemment décrit (exemple 2). On opère la purification par une double recristallisation dans l'hexane industriel.

On obtient 2,5 g (87,8 %) de N,N-diméthyldithiocarbamate de diacétone galactose pur d'après les analyses HPLC, RMN$^1$H, RMN$^{13}$C.

F = 128-129° C ; $[\alpha]_D^{20}$ = -10,3° ($CHCl_3$).

Exemple 4 :

Synthèse des désoxy-6 N,N diméthyl dithiocarbamate et désoxy N,N diéthyl dithiocarbamate di-O-isopropylidène 1,2 : 3,4-α-D galactopyrannose à partir du tosylate-6 di-O-isopropylidène 1,2 : 3,4 α-D galactopyrannose (tosylate de diacétone galactose) et des dithiocarbamates de lithium correspondants.

les N,N diméthyl dithiocarbamate et N,N diéthyl dithiocarbamate de lithium (DMDTC-Li et DEDTC-Li) sont préalablement préparés comme suit : à une solution contenant 11 g de $Li_2\ SO_4$ et 45 g de DEDTC-Na, 3 $H_2O$ dans 110 ml d'eau, agitée pendant 30 mn, on ajoute 500 ml d'éthanol/eau 95/5 (v/v). Le précipité de $Na_2\ SO_4$ est écarté par filtration. Le filtrat évaporé sous pression réduite puis séché pendant 12 heures en dessicateur en présence de $P_2O_5$ donne 34 g de DEDTC-Li de pureté supérieure à 90 % d'après contrôle par photométrie de flamme. Une pureté supérieure à 99 % a été obtenue en faisant passer le filtrat précédent sur colonne échangeuse d'ions (saturée d'ions lithium).

Une solution contenant 11 g de $Li_2SO_4$ et 35,8 g de DMDTC - Na,2$H_2O$ traitée dans les conditions ci-dessus donne 29,8 g de DMDTC-Li de pureté supérieure à 90 %. La pureté supérieure à 99 % a été obtenue après passage du filtrat sur colonne échangeuse d'ions.

Préparation du désoxy-6 N,N diéthyl dithiocarbamate-6 di-O-isopropylidène 1,2 : 3,4 α-D galactopyrannose.

0,10 mol de tosylate de diacétone galactose et 0,13 mol de DEDTC-Li sont placés dans un ballon contenant 450 ml de solvant toluène/HMPA 66/34 (v/v), après 2 heures de réaction sous agitation à 110° C, extraction et purification, le produit est obtenu avec un rendement de 94 %.

Préparation du désoxy-6 N,N diméthyl dithiocarbamate-6 di-O-isopropylidène 1,2 : 3,4 α-D galactopyrannose.

0,10 mol de tosylate de diacétone galactose et 0,13 mol de DMDTC-Li sont traités comme ci-dessus. Après purification le produit est obtenu avec un rendement de 93,5 %.

Exemple 5 :

Synthèse du désoxy-3 N,N diéthyldithiocarbamate di-O-isopropylidène-1,2 : 5,6-α -D glucofurannose à partir du désoxy-3 iodo-3 di-O-isopropylidène- 1,2: 5,6-α-D allofurannose.

10 g ($2,7.10^{-2}$ mol) de désoxy-3 iodo-3 di-O-isopropylidène-1,2:5,6-α-D allofurannose et 12,18 g ($5,4.10^{-2}$ mol) de N,N-diéthyldithiocarbamate de sodium trihydraté (DEDTC-Na) sont placés dans un tricol de 250 ml contenant 150 ml d'hexaméthyl phosphorotriamide (HMPA). Sous agitation, le mélange est porté à 60° C. La réaction est arrêtée après 24 heures de réaction, l'avancement étant de 95 % d'après contrôle HPLC.

On additionne, au milieu réactionnel, un mélange hexane/éther éthylique 90/10 (V/V), puis on lave à l'eau glacée. La phase aqueuse est séchée sur sulfate de sodium anhydre, puis évaporée sous pression réduite. On obtient 18 g de brut.

On opère la purification par passage sur colonne de silice ; après élution avec un mélange hexane/éther éthylique 85/15 (V/V), on isole 8,6 g (87 %) de N,N-diéthyldithiocarbamate de diacétone glucose pur d'après les analyses HPLC, RMN[1]H, RMN[13]C.
F = 83-84° C ; $[\alpha]_D^{20}$ = -44,3° ($CHCl_3$).

On donne dans les tableaux V et VI respectivement les caractéristiques des spectres RMN[1]H et RMN[13]C du DEDTC de diacétone glucose obtenu.

En remplaçant le solvant HMPA pur par un mélange acétone / HMPA 50/50 (V/V), on obtient après 48 heures, un taux de conversion du iodo diacétone allose de 85 %.

En remplaçant le solvant HMPA par le mélange toluène/HMPA (50/50) (v/v), on obtient après 18 heures à 110° C un taux de conversion de l'iodo diacétone allose de 96%.

Les diméthyls-sont obtenus par les sels de DMDTC.

En opérant dans le mélange toluène/HMPA (50/50) (v/v) et en remplaçant DEDTC-Na par le sel de lithium correspondant on obtient après 2 heures à 110° C un taux de conversion de l'iodo diacétone allose de 85 %

En prenant le trifluorométhyl sulfonate-3 di-O-isopropylidène 1,2:5,6 α-D glucofurannose à la place de l'iodo-3 diacétone allose et en opérant dans le mélange HMPA/toluène (50/50) (v/v) avec DEDTC-Li en excès de 30 %, on obtient le désoxy-3 diéthyl dithiocarbamate-3 di-O-isopropylidène 1,2 : 5,6 α-D allofurannose.

Exemple 6 :

Préparation du désoxy-1 N,N diéthyl dithiocarbamate di-O-isopropylidène 2,3 α-D fructopyrannose (DEDTC de diacétone fructose):

0,1 mol de désoxy-1 iodo-1 di-O-isopropylidène 2,3 : 4,5 α-D fructopyrannose (iodo diacétone fructose) et 0,13 mol de DEDTC-Na sont placés dans un ballon contenant 250 ml d'HMPA. Après 8 heures de réaction à 70° C le taux de conversion du iodo diacétone fructose est de 88 %, après extraction et purification le produit est obtenu avec un rendement de 74%.
$[\alpha]_D^{20}$ = - 5,02° (CH $Cl_3$) F = 100° C.

Les spectres RMN $^1$H et $^{13}$C sont donnés dans les tableaux VII et VIII

En opérant avec le mélange HMPA/toluène (50/50) (v/v) à 110° C, le taux de conversion de l'iodo diacétone fructose est de 82 % après 18 heures de réaction.

Exemple 7 :

Produits obtenus par déprotection des sites hydroxylés.

La déprotection des produits

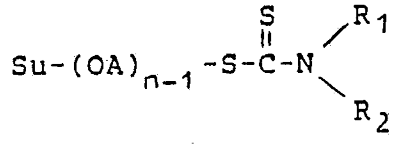

a été réalisée dans l'éthanol/eau,95/5 (V/V) par acidocatalyse soit en phase hétérogène sur colonne AMBERLIST H+ Wet (ROHM et HAAS), soit en phase homogène en présence de $H_2SO_4$ à la concentration 0,2 N.

- Préparation en phase hétérogène du désoxy-1 N,N-diéthyl dithiocarbamate-1 glycérol (DEDTC-Glycérol) à partir du désoxy-1 N,N-diéthyl dithiocarbamate-1 O-isopropylidène-2,3 glycérol (DEDTC -Solkétal) et du désoxy-1 N,N diméthyl dithiocarbamate-1 glycérol (DMDTC-glycérol) à partir du désoxy-1 N,N diméthyl

dithiocarbamate-1 O-isopropylidène 2,3 glycérol (DMDTC solkétal).

On fait passer à un débit de 2 ml/mn, une solution de 10 g de DEDTC-Solkétal dans 100 ml d'alcool à 95°, sur la colonne thermostatée à 65° C, contenant 100 g de résine AMBERLIST H+. On rince la colonne avec 100 ml d'alcool à 95°. Après évaporation du solvant et purification sur gel de silice, 50/50 (V/V) on obtient 8,4 g de DEDTC-glycérol (rendement 92 %), liquide à la température ambiante, pur d'après les analyses HPLC, RMN $^1H$ et $^{13}C$.
$[n]^D_{20}$ = 1,5803.

On donne dans les tableaux IX et X respectivement les caractéristiques des spectres RMN$^1H$ et RMN $^{13}C$ du DEDTC de glycérol obtenu.

Dans les mêmes conditions, on obtient le DMDTC-glycérol à partir du DMDTC-solkétal avec un rendement de 88 %.

Préparation du di-O-butanoyl=2,3 désoxy-1 N,N diéthyl dithiocarbamate-1 glycérol (dibutyrate de DEDTC glycérol).

On place dans un ballon 1,5.10$^{-2}$ mol de DEDTC-glycérol dans 15 ml d'un mélange pyridine/toluène (50/50) (V/V) et on ajoute goutte à goutte sous agitation 3,0.10$^{-2}$ mol de chlorure de butyryle dissous dans 10 ml de toluène ; après 5 heures de réaction à température ambiante, le mélange réactionnel est lavé une fois avec une solution aqueuse d'HCl 1 N et deux fois à l'eau, la phase organique est séchée sur $Na_2SO_4$ puis évaporée, le résidu est purifié sur gel de silice avec le mélange acétone/hexane industriel comme éluant. Après purification, on obtient un rendement de 80 %.
$[n]^{20}$ = 1,5142

Les spectres RMN $^1H$ et $^{13}C$ sont donnés dans les tableaux XI et XII.

Préparation du di-O-palmitoyl-2,3 désoxy-1 N,N diéthyl dithiocarbamate-1 glycérol (dipalmitate de DEDTC glycérol).

On opère dans les mêmes conditions que pour le dibutyrate de DEDTC glycérol. Le résidu, après extraction et évaporation du solvant, est purifié par recristallisation dans l'hexane industriel.

On obtient un rendement de 88 %.

le spectres RMN $^1H$ et $^{13}C$ sont donnés dans les tableaux XV et XVI.

- Préparation en phase hétérogène du désoxy-3 N,N diéthyl dithiocarbamate-3 O-isopropylidène-1,2 α-D-glucofurannose (DEDTC-MAGlu) et du désoxy-3 N,N diéthyl dithiocarbamate-3 D-glucopyrannose (DEDTC-Glucose) à partir du désoxy-3 N,N diéthyl dithiocarbamate-3 di O-isopropylidène 1,2 : 5,6α-glucofurannose (DEDTC-DAGlu).

La déprotection des sites hydroxylés par acido-catalyse sur colonne de résine $H^+$ a été conduite dans les mêmes conditions de solvant, de concentration, de débit et de température que précédemment. Après rinçage de la colonne par de l'alcool à 95° et évaporation du solvant, on obtient, à partir de 10 g de produit initial, 8,5 g de brut. La chromatographie sur gel de silice (60 g) permet de recueillir 3 fractions :
- 0,58 g (5,8 %) de produit de départ, élué avec le mélange hexane/acétone, 85/15 (V/V).
- 6,8 g (75 %) de DEDTC-MAGlu, élué avec le mélange hexane/acétone, 70/30 (V/V), pur d'après les analyses HPLC, RMN $^1H$ $^{13}C$.
F = 78-80° C, $[\alpha]^{22}_D$ = + 90,8° ($CHCl_3$)
- 0,6 g (7,5 %)- de DEDTC-Glucose, élué à l'acétone seule, pur d'après l'analyse HPLC.

Pour une concentration en substrat de départ et une quantité de résine données, la proportion de dérivé totalement débloqué (DEDTC-Glucose) peut être augmentée aux dépens du dérivé monodébloqué (DEDTC-MAGlu) en diminuant le débit et/ou en augmentant la température par exemple à 70° C avec un débit de 0,5 ml/mn, on obtient la disparition complète du produit de départ et les produits DEDTC-MAGlu et DEDTC-Glucose dans les proportions 30/70.

Les mêmes conditions opératoires ont permis de préparer le désoxy-3 N,N diméthyl dithiocarbamate-3 O-isopropylidène 1,2 α-D glucofurannose (DMDTC-MAGlu) et le désoxy-3 N,N diméthyl dithiocarbamate-3 D-glucopyrannose (DMDTC-glucose) à partir du DMDTC-diacétone glucose, avec des rendements similaires.

- Préparation en phase homogène, en présence de $H_2SO_4$du DEDTC-MAGlu et du DEDTC-Glucose à partir du DEDTC-DAGlu.

La déprotection des sites hydroxylés par acido-catalyse en présence de $H_2SO_4$ 0,2 N a été conduite dans l'éthanol à 95°, à la température contrôlée de 50°C, à partir de 10 g de DEDTC-DAGlu (25,5 mmol) dans 100 ml de solvant, sous agitation. Après une heure, 96 % de substrat ayant réagi, on neutralise à pH 7 par de la lessive de soude 8 N. On filtre le sulfate de sodium formé sur fritté n° 4 et le filtrat évaporé donne 8,3 g de produit brut qui est chromatographié comme précédemment, pour donner :

- 0,35 g (3,5 %) de produit de départ ;
- 6,3 g (70 %) de DEDTC-MAGlu pur ;
- 0,5 g (6 %) de DEDTC-Glu pur.

En poursuivant pendant 20 heures l'acido-catalyse dans les mêmes conditions de température, solvant et acidité, la déprotection des sites hydroxylés du DEDTC-DAGlu est achevée à plus de 90 %, mais le pourcentage de DEDTC-Glu n'excède pas 60 %, car une telle durée de réaction provoque la dégradation du produit par le solvant.

On donne dans les tableaux XV et XVI respectivement les caractéristiques des spectres RMN$^1$H et RMN $^{13}$C du DEDTC de monoacétone glucose (DEDTC-MAGlu) obtenu.

Il est donné dans le tableau XVII les caractéristiques du spectre RMN $^{13}$C du DEDTC de glucose obtenu.

Exemple 8 :

Préparation en phase pseudohomogène des désoxy-6 N,N diéthyl dithiocarbamate D-galactopyrannose (DEDTC galactose) et désoxy-6 N,N diméthyl dithiocarbamate D-galactopyrannose (DMDTC-galactose).

On verse, dans un ballon contenant 40 ml de dioxanne et 10 ml HCl aqueux 12 N , $10^{-2}$M de DEDTC-diacétone galactose. Après 1 heure d'agitation à 50°C la solution devient limpide et la totalité du DEDTC-diacétone galactose a disparu d'après les contrôles par C.C.M. On neutralise alors en additionnant lentement de la triéthylamine (TEA) et l'on sépare le précipité de chlorhydrate de TEA par filtration. Le filtrat est évaporé puis repris à l'eau pour précipiter le produit attendu. On obtient le DEDTC-galactose avec un rendement de 60%. Le spectre RMN $^{13}$C est donné dans le tableau XVIII.

Le DMDTC-galactose a été obtenu dans les mêmes conditions opératoires avec un rendement de 58%.

Ce même procédé s'applique à la préparation des désoxy-1 N,N diéthyl (diméthyl) dithiocarbamate du glucose, du fructose, du mannose ou d'autres monosaccharides à partir des précurseurs di-O-isopropylidène.

Exemple 9 :

On compare les propriétés biologiques (DL50 chez la souris) des esters dithiocarbamiques décrits dans les exemples 2, 3, 4, 5 et 8 à celles du diéthyldithiocarbamate salin DEDTC-Na disponible dans le commerce.

Les résultats obtenus sont rassemblés dans le tableau XIX.

Il ressort de ces résultats que les esters de l'acide dithiocarbamique conformes à l'invention présentent une toxicité nettement moins grande que le DEDTC-Na disponible dans le commerce.

Par ailleurs, il s'est avéré que les produits intermédiaires dithiocarbamates de lithium, notamment diméthyldithiocarbamate de lithium et de diéthyldithiocarbamate de lithium sont très actifs, peu toxiques et peuvent être utilisés purs ou associés, comme médicaments pour les mêmes applications, par exemple à des doses similaires à celles utilisées dans le cas du DEDTC de sodium.

L'utilisation des dithiocarbamates de lithium dans le procédé de préparation d'esters selon l'invention est préférée à celle des autres dithiocarbamates salins car les réactions sont plus rapides à une température plus basse, et on peut utiliser une gamme de solvants plus large.

TABLEAU I

SPECTRE RMN $^1$H DU DEDTC DE SOLKETAL

| Formule | Protons | Nature du signal | δ en ppm/TMS dans $CDCl_3$ | Constantes de couplage (I) |
|---|---|---|---|---|
| 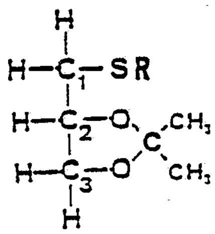 | H-1<br>H-1' | d | 3,58 | J 1-2 =5,9 |
| | H-2 | m | 4,41 | J 2-3 =6,1 |
| | H-3 | dd | 4,10 | J 3-3'=8,4 |
| | H-3' | dd | 3,74 | |
| R = 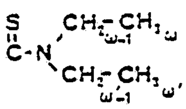 | $CH_3$ i | s | 1,44 | |
| | $CH_3$ i' | s | 1,34 | |
| | $CH_2$( Ω-1) | m | 3,98 | J = 7,3 |
| | $CH_2$(Ω'-1) | m | 3,68 | J = 7 |
| | $CH_3$ Ω | t | 1,30 | J = 7,2 |
| | $CH_3$ Ω' | t | 1,27 | J = 7,1 |

TABLEAU II

SPECTRE RMN $^{13}C$ DU DEDTC DE SOLKETAL

| Formule | Carbones | δ en ppm/TMS dans $CDCl_3$ |
| --- | --- | --- |
| 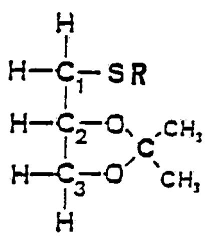 | C - 1 | 39,99 |
| | C - 2 | 74,44 |
| | C - 3 | 66,42 |
| R= 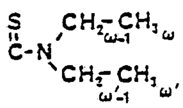 | 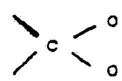 | 109,29 |
| | $2CH_3$(isoprop.) | 26,61-25,40 |
| | $CH_2$ (Ω-1) | 49,67 |
| | $CH_2$ (Ω'-1) | 46,64 |
| | $CH_3$( Ω) | 12,42 |
| | $CH_3$(Ω') | 11,41 |
| | $CS_2$ | 194,51 |

TABLEAU III

SPECTRE RMN $^1$H DU DEDTC DIACETONEGALACTOSE (DEDTC-DAGal)

| Formule | Protons | Nature du signal | δ en ppm/TMS dans $CDCl_3$ | Constantes de couplage (I) |
|---|---|---|---|---|
| 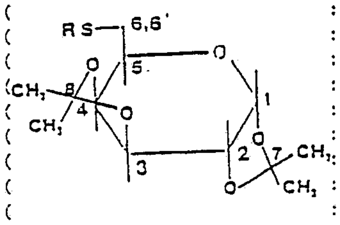 | H-1 | d | 5,51 | J 1-2 = 5 |
| | H-2 | q | 4,29 | J 2-3 = 2,4 |
| | H-3 | q | 4,62 | J 3-4 = 7,95 |
| | H-4 | q | 4,24 | J 4-5 = 1,8 |
| | H-5 | hex | 3,92 | J 5-6 = 6,6 |
| | H-6 | q | 3,31 | J 6-6' |
| | H-6' | | 3,63 | J 5-6 = 6,8 |
| R= 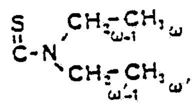 | 4$CH_3$ | s | 1,31 | |
| | | s | 1,36 | |
| | | s | 1,47 | |
| | (isoprop.) | s | 1,48 | |
| | $CH_2$( Ω-1) | q | 3,72 | |
| | $CH_2$(Ω'-1) | q | 4,08 | |
| | $CH_3$( Ω) | t | 1,24 | |
| | $CH_3$(Ω') | t | 1,29 | J Ω — Ω-1 =7 |

TABLEAU IV

SPECTRE RMN $^{13}C$ DU DEDTC DE DIACTEONEGALACTOSE

| Formule | Carbones | δ en ppm/TMS dans $CDCl_3$ |
|---|---|---|
| 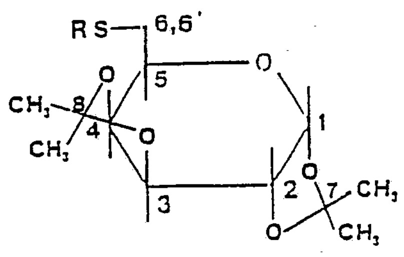 | C-1 | 96,48 |
| | C-2 | 70,90 |
| | C-3 | 70,56 |
| | C-4 | 66,76 |
| | C-5 | 72,37 |
| | C-6 | 36,88 |
| | C-7 | 109,27 |
| | C-8 | 108,78 |
| 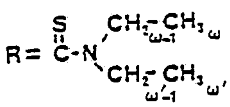 | $4CH_3$(isoprop.) | 24,37-25,05-25,90 25.90 |
| | $CS_2$ | 195,27 |
| | $CH_2$( Ω-1) | 49,38 |
| | $CH_2$(Ω'-1) | 46,70 |
| | $CH_3$ Ω | 12,45 |
| | $CH_3$ Ω' | 11,43 |

TABLEAU V

SPECTRE RMN[1]H DU DEDTC DIACETONEGLUCOSE (DEDTC-DAGlu)

| Formule | Protons | Nature du signal | δ en ppm/TMS dans $CDCl_3$ | Constantes de couplage (J) |
|---|---|---|---|---|
| 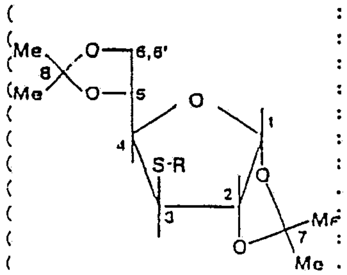 | H-1 | d | 5,75 | J 1-2 = 3,2 |
| | H-2 | d | 4,61 | J 2-3 = 0,6 |
| | H-3 | dd | 4,70 | J 3-4 = 3,7 |
| | H-4 | dd | 4,37 | J 4-5 = 8,1 |
| | H-5 | m | 4,19 | J 5-6 |
| | H-6 | q | 4,01 | J 6-6' = 8,6 |
| R = 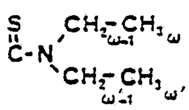 | H-6' | | 3,95 | J 5-6' |
| | 4$CH_3$ (isoprop.) | s | 1,26 | |
| | | s | 1,26 | |
| | | s | 1,35 | |
| | | s | 1,49 | |
| | $CH_2$(Ω-1) | m | 3,69 | |
| | $CH_2$(Ω'-1) | m | 3,98 | |
| | $CH_3$(Ω) | t | 1,20 | |
| | $CH_3$(Ω') | t | 1,26 | J Ω —Ω'-1 = 6,4 |

TABLEAU VI

SPECTRE RMN$^{13}$C DU DEDTC DE DIACETONEGLUCOSE (DEDTC-DAGlu)

| Formule | Carbones | δ en ppm/TMS dans $CDCl_3$ |
|---|---|---|
| 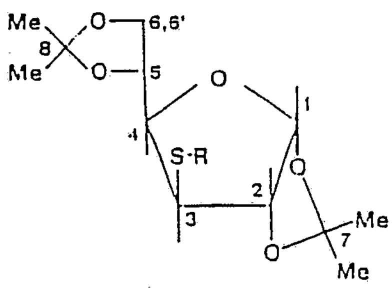 | C-1 | 104,47 |
| | C-2 | 86,69 |
| | C-3 | 57,12 |
| | C-4 | 78,96 |
| | C-5 | 73,97 |
| | C-6 | 67,44 |
| R = 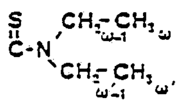 | C-7 | 112,12 |
| | C-8 | 109,45 |
| | $4CH_3$ (Isoprop.) | 26,29-26,52-25,20-26,74 |
| | $CH_2$(Ω-1) | 49,29 |
| | $CH_2$(Ω'-1) | 46,63 |
| | $CH_3$(Ω) | 12,44 |
| | $CH_3$(Ω') | 11,43 |
| | $CS_2$ | 191,92 |

TABLEAU VII

SPECTRE DE RMN $^1$H DU DEDTC-1 DE DIACETONE FRUCTOSE

| FORMULE | Protons | Nature du signal | δ en ppm/TMS dans $CDCl_3$ | Constantes de couplage (I) |
|---|---|---|---|---|
| | H1 H1' | d | 3,81 | J1,2=1,2Hz |
| | H3 | d | 4,29 | J3-4=2,5 |
| | H4 | dd | 4,52 | J4-3=2,4 |
| | | | | J4-5=7,9 |
| | H5 | dd | 4,16 | J5-6=1,9 |
| | | | | J5-4=7,9 |
| 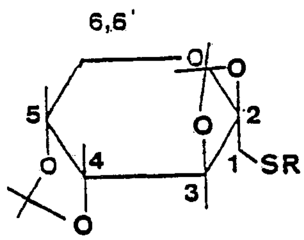 | H6 | dd | 3,81 | J5-6=1,9 |
| | | | | J5-6'=0,7 |
| | H6' | dd | 3,69 | J6-6'=13,5 |
| | $2CH_3$ | s | 1,46 | |
| | $CH_3$ | s | 1,39 | |
| R = 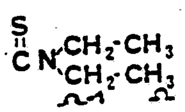 | $CH_3$ | s | 1,28 | |
| | $CH_3-CH_2$ | m | 1,23 | JΩ-(Ω-1) = non |
| | | | 1,21 | calculable |
| | $CH_3-CH_2$ | m | 3,92 | JΩ-(Ω-1) = non |
| | | | | calculable |

TABLEAU VIII

SPECTRE DE RMN $^{13}C$ DU DEDTC-1 DE DIACETONE FRUCTOSE

| FORMULE | CARBONE | δ en ppm/TMS dans $CDCl_3$ |
|---|---|---|
| | $C_1$ | 46,49 |
| | $C_2$ | 102,68 |
| | $C_3$ | 73,16 |
| | $C_4$ | 70,43 |
| | $C_5$ | 70,43 |
| | $C_6$ | 61,39 |
| 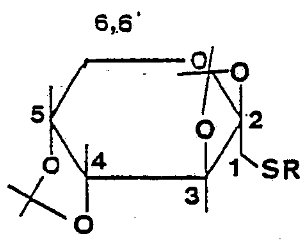 | | 109,01 |
| | | 108,38 |
| | $CH_3$ | 26,29 |
| | $CH_3$ | 25,88 |
| | $CH_3$ | 25,08 |
| R = 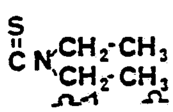 | $CH_3$ | 24,04 |
| | $CH_3$-$CH_2$ | 12,50 |
| | | 11,47 |
| | $CH_3$-$CH_2$ | 49,67 |
| | | 46,69 |
| | $CS_2$ | 195,29 |

TABLEAU IX

SPECTRE RMN $^1H$ du DEDTC GLYCEROL

| Formule | Protons | Nature du signal | $\delta$ en ppm/TMS dans $CDCl_3$ | Constantes de couplage (I) |
|---|---|---|---|---|
| 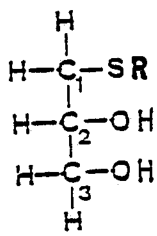 | H-1<br>H-1' | m | 3,53-3,66 | J 1-1'<br>J 1-2 |
| | H-2 | m | 3,9 | J 1'-2 |
| | H-3 | dd | 3,44 | J 2-3 |
| | H-3' | dd | 3,49 | J 2-3' |
| R = 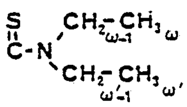 | $CH_2(\Omega-1)$ | q | 3,98 | J = 7,1 |
| | $CH_2(\Omega'-1)$ | q | 3,73 | |
| | $CH_3$ Ω | t | 1,26 | |
| | $CH_3$ Ω' | t | 1,22 | |

TABLEAU X

SPECTRE RMN $^{13}C$ DU DEDTC GLYCEROL

| Formule | Carbones | δ en ppm/TMS dans $CDCl_3$ |
| --- | --- | --- |
| 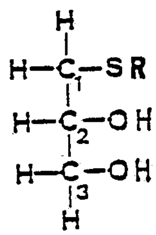 | C-1 | 39,06 |
| | C-2 | 71,36 |
| | C-3 | 64,51 |
| 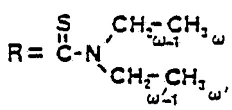 | $CH_2(\Omega-1)$ | 50,16 |
| | $CH_2(\Omega'-1)$ | 47,07 |
| | $CH_3$ Ω | 12,38 |
| | $CH_3$ Ω' | 11,47 |
| | $CS_2$ | 195,75 |

TABLEAU XI

SPECTRE RMN $^{1}H$ DU DIBUTYRATE DEDTC-GLYCEROL

| FORMULE | Protons | Nature du signal | δ en ppm/TMS dans $CDCl_3$ | Constantes de couplage (I) |
|---|---|---|---|---|
| 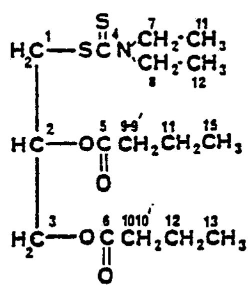 | H-1 | dd | 3,63 | J1-1'=14,2<br>J1-2 =4,9 |
| | H-1' | dd | 3,35 | J1-1'=14,1<br>J1'-2=7,4 |
| | H2 | m | 5,17 | |
| | H-3 | dd | 4,23 | J3-3'=11,9<br>J3-2=3,5 |
| | H-3' | dd | 4,02 | J3-2=3,5 |
| | H-7,7' | qb | 3,86 | J=6,8 |
| | H-8,8' | qb | 3,59 | J=6,8 |
| | H-9,9' | t | 2,15 | J=7,39 |
| | H-10,10' | t | 2,14 | J=7,31 |
| | H-11,11',12,12' | h | 1,50 | J=7,38 |
| | $CH_3$(13) , $CH_3$(14) | m | 1,13 ; 1,11 | J=6,81 |
| | $CH_3$(15) , $CH_3$(16) | m | 0,795 | J=7,31 ; 7,35 |

TABLEAU XII

SPECTRE DE RMN $^{13}C$ DU DIBUTYRATE DE DEDTC GLYCEROL

| FORMULE | CARBONE | δ en ppm/TMS dans $CDCl_3$ |
|---|---|---|
| | $C_1$ | 37,23 |
| | $C_2$ | 69,83 |
| | $C_3$ | 63,68 |
| | $C_6=0$ | 172,85 |
| | $C_5=0$ | 172,34 |
| 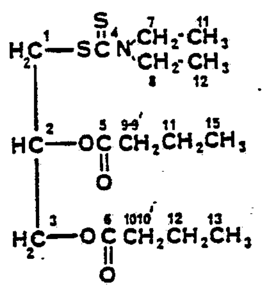 | $C_4$ | 193,67 |
| | $CH_2$ (7) | 49,76 |
| | $CH_2$ (8) | 46,64 |
| | $CH_2$ (9) | 35,95 |
| | $CH_2$ (10) | 35,78 |
| | $CH_2$ (11) | 18,22 |
| | $CH_2$ (12) | |
| | $CH_3$ (13) | 12,36 |
| | $CH_3$ (14) | 11,34 |
| | $CH_3$ (15) | 13,46 |
| | $CH_3$ (16) | |

TABLEAU XIII

SPECTRE RMN $^1H$ DU DIPALMITATE DE DEDTC-GLYCEROL

| FORMULE | Protons | Nature du signal | δ en ppm/TMS dans $CDCl_3$ | Constantes de couplage (I) |
|---|---|---|---|---|
| 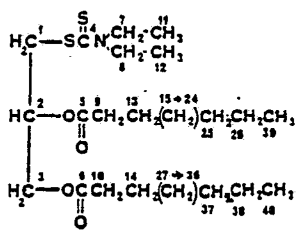 | H-1 | dd | 3,74 | J1-1'=14,0<br>J1-2=5,0 |
| | H-1' | dd | 3,46 | J1'-1=14,2<br>J1'-2=7,4 |
| | H-2 | m | 5,28 | |
| | H-3 | dd | 4,33 | J3-3'=12,1<br>J3-2=3,6 |
| | H-3' | dd | 4,13 | J3'-3=12,0<br>J3'2=5,7 |
| | H-7 et H-7' | qb | 3,97 | J=7,0 |
| | H-8 et H-8' | qb | 3,69 | J=6,8 |
| | H-9 et H-9' | m | 2,27 | J=7,5 |
| | H-10 et H-10' | m | 2,26 | J=7,4 |

TABLEAU XIV

SPECTRE DE RMN $^{13}C$ DU DIPALMITATE DE DEDTC-GLYCEROL

| FORMULE | CARBONE | δ en ppm/TMS dans $CDCl_3$ |
|---|---|---|
| | $C_1$ | 37,39 |
| | $C_2$ | 69,95 |
| | $C_3$ | 63,84 |
| | $C_6$=0 | 173,29 |
| | $C_5$=0 | 172,74 |
| 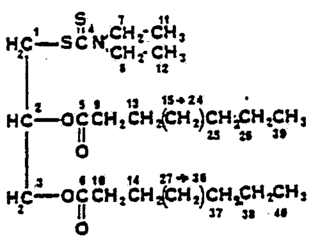 | $C_4$=S | 193,90 |
| | $CH_2$ (9) | 34,25 |
| | $CH_2$ (10) | 34,87 |
| | $CH_2$ (13 et 14) | 31,85 |
| | $CH_2$ (15 à 24 et 27 à 36) | 29,61 à 28,95 |
| | $CH_2$ (25 et 37) | 24,87 |
| | $CH_2$ (26 et 38) | 22,62 |
| | $CH_3$ (39 et 40) | 14,04 |
| | $CH_2$ (7) | 49,87 |
| | $CH_2$ (8) | 46,69 |
| | $CH_3$ (11) | 12,45 |
| | $CH_3$ (12) | 11,45 |

TABLEAU XV

SPECTRE RMN $^1H$ DU DEDTC-3 MONOACETONE GLUCOSE (DEDTC-MAGlu)

| Formule | Protons | Nature du signal | δ en ppm/TMS dans $CDCl_3$ | Constantes de couplage (I) |
|---|---|---|---|---|
| 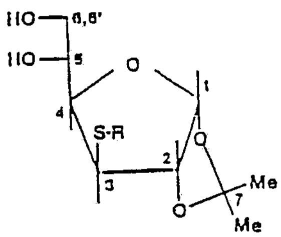 | H-1 | d | 5,73 | J 1-2 = 3,6 |
| | H-2 | d | 4,73 | J 2-3 = 0,6 |
| | H-3 | dd | 4,83 | J 3-4 = 3,4 |
| | H-4 | dd | 4,35 | J 4-5 8,8 |
| | H-5 | | | J 5-6 |
| | H-6 | m | 3,55-3,87 | J 6-6' |
| | H-6' | | | J 5-6' |
| R= 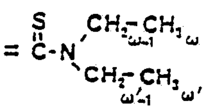 | $2CH_3$ | s | 1,26 | |
| | (isoprop.) | s | 1,49 | |
| | $CH_2(\Omega-1)$ | qd | 3,93 | |
| | $CH_2(\Omega'-1)$ | qd | 4,00 | |
| | $CH_3(\Omega)$ | t | 1,23 | |
| | $CH_3(\Omega')$ | t | 1,26 | $J\Omega - \Omega-1 = 7,1$ |
| | $OH_5$ | s | 3,46 | |
| | $OH_6$ | s | 2,51 | |

TABLEAU XVI

SPECTRE RMN $^{13}C$ DU DEDTC-3 MONO ACETONE GLUCOSE (DEDTC-MAGlu)

| Formules | Carbones | δ en ppm/TMS dans $CDCl_3$ |
|---|---|---|
| 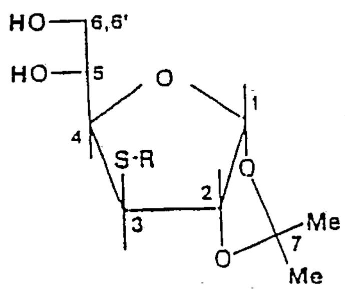 | C -1 | 104,47 |
| | C -2 | 85,86 |
| | C -3 | 58,69 |
| | C -4 | 78,98 |
| | C -5 | 70,30 |
| | C -6 | 64,13 |
| 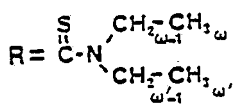 | C -7 | 112,26 |
| | $2CH_3$(isoprop.) | 26,24-26,42 |
| | $CH_2$( Ω-1) | 47,41 |
| | $CH_2$(Ω'-1) | 50,34 |
| | $CH_3$( Ω) | 11,46 |
| | $CH_3$(Ω') | 12,49 |
| | $CS_2$ | 192,73 |

TABLEAU XVII

SPECTRE RMN $^{13}C$ DU DEDTC DE GLUCOSE

| Formule | Carbones | δ en ppm/TMS dans $CDCl_3$ |
|---|---|---|
| 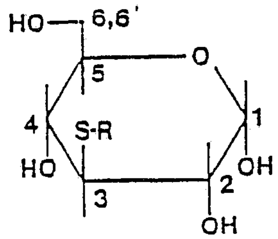 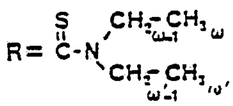 | $C_1$ | 91,77 α<br>98,11 β |
| | $C_2$ | 70,72<br>70,14 (α, β) |
| | $C_3$ | 57,38<br>59,80 (α, β) |
| | $C_4$ | 69,93 |
| | $C_5$ | 73,15<br>72,44 (α, β) |
| | $C_6$ | 62,18<br>61,86 |
| | $CS_2$ | 196,36<br>195,99 (α,β) |
| | $CH_2(\Omega-1)$ | 50,37 |
| | $CH_2(\Omega'-1)$ | 47,84 |
| | $CH_3$ Ω | 11,62 |
| | $CH_3$ Ω' | 12,57 |

TABLEAU XVIII

SPECTRE DE RMN $^{13}C$ DE DEDTC-6 DE GALACTOSE

| FORMULE | CARBONE | δ en ppm/TMS dans $CDCl_3$ |
|---|---|---|
| | Forme β | |
| | $C_1$ | 99,36 |
| | $C_2$ | 74,52 |
| | $C_3$ | 75,57 |
| | $C_4$ | 72,53 |
| | $C_5$ | 76,48 |
| 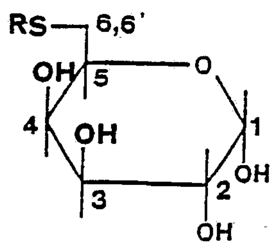 | $C_6$ | 39,39 |
| | C = S | 197,33 |
| | Forme α | |
| | $C_1$ | 95,24 |
| | $C_2$ | 73,04 |
| 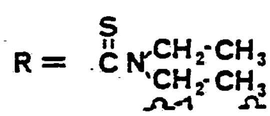 | $C_3$ | 71,01 |
| | $C_4$ | 72,53 |
| | $C_5$ | 71,96 |
| | $C_6$ | 39,51 |
| | C= S | 197,33 |
| | Formes β et α | |
| | $CH_2$ | 53,16 |
| | $CH_2'$ | 50,55 |
| | $CH_3$ | 14,25 |
| | $CH_3'$ | 13,64 |

TABLEAU XIX

| DL50 chez la souris | | | |
|---|---|---|---|
| | SOLVANT | VOIE ORALE (g/kg) | VOIE INTRA PERITONEALE (g/kg) |
| DEDTC-Na commercial | eau<br>DMSO | 4,00 ± 0,32<br>1,90 ± 0,34 | 1,5<br>1,15 ± 0,01 |
| DEDTC diacétone glucose | DMSO | > 8 | 4,20 ± 0,48 |
| DEDTC diacétone galactose | DMSO | > 8 | 2,65 ± 0,53 |
| DMDTC diacétone galactose | DMSO | > 8 | 2,15 ± 0,35 |
| DMDTC glycérol | DMSO | 4,00 ± 0,30 | 1,4 ± 0,28 |
| Dipalmitate DEDTC glycérol | DMSO | > 8 | 4,2 ± 0,46 |
| Dibutyrate DEDTC glycérol | DMSO | > 8 | 3,5 ± 0,42 |

**Revendications**

1. Procédé de préparation d'esters d'acides dithiocarbamiques de formule

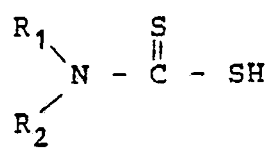

dans laquelle $R_1$ et $R_2$ sont des radicaux alkyle saturés ou non, substitués ou non avec des groupements carbonés ou des hétéroatomes ou encore formant avec l'atome d'azote un cycle saturé ou non comportant ou non des hétéroatomes à partir d'alcools ou de polyols, caractérisé en ce que l'on transforme, dans une étape préliminaire, au moins un groupement hydroxylé de la molécule mono- ou polyhydroxylée en un groupement partant (Y) et en ce que l'on déplace le groupement partant (Y) par l'anion

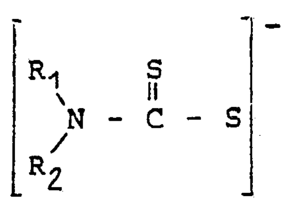

$R_1$ et $R_2$ ayant la signification précitée, introduit sous forme de sel dans le milieu approprié, et en ce que le groupement partant Y peut être un iodure ou un tosylate ou un brosylate ou un triflate ou un imidazole sulfonate qui, notamment dans le cas de mono- ou di-saccharides, peut être greffé sur un site secondaire autre qu'anomérique aussi bien que sur un site primaire ou anomérique.

2. Procédé selon la revendication 1, caractérisé en ce que, lorsqu'il est appliqué à la préparation d'un monoester d'acide dithiocarbamique à partir de polyols, notés [Su $(OH)_n$] , le procédé comprend les étapes successives :
- de protection sélective de (n-1) groupements OH pour donner [Su $(O\text{-}A)_{n-1}$-OH)], de préférence sous forme de dioxolanne,
- de transformation du groupement OH en un groupement partant (Y) pour donner [Su $(O\text{-}A)_{n-1}$-Y], noté [Su P-Y]
- d'estérification par action d'un sel, de préférence de lithium, de l'acide dithiocarbamique

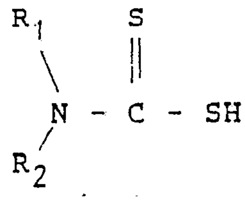

sur [SU $(O\text{-}A)_{n-1}$ -Y], noté [SU P-Y] conduisant à

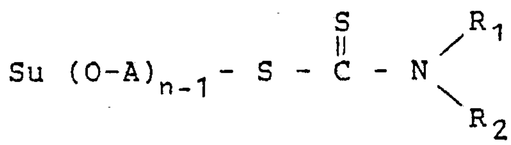

- et de regénération partielle ou totale des sites hydroxylés bloqués.

3. Procédé selon l'une quelconque des revendications 1 et 2, caractérisé en ce que le groupement partant (Y) est constitué par un halogénure.

4. Procédé selon la revendication 3, caractérisé en ce que l'halogénure est l'iodure.

5. Procédé selon l'une quelconque des revendications 1 et 2, caractérisé en ce que le groupement partant (Y) est constitué par un sulfonate.

6. Procédé selon la revendication 5, caractérisé en ce que le sulfonate est choisi dans le groupe constitué par les brosylates, tosylates, triflates, imidazole sulfonates.

7. Procédé selon l'une quelconque des revendications 1 à 6, caractérisé en ce que la préparation de l'ester dithiocarbamique comporte les étapes suivantes :

- de mise en contact de l'intermédiaire [Su P-Y] et d'un sel de l'acide dithiocarbamique

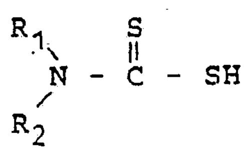

en présence d'un solvant aprotique polaire, le sel de l'acide dithiocarbamique étant utilisé en excès par rapport à la quantité stoechiométrique,
- de réaction du mélange, sous agitation, le temps nécessaire pour avoir un taux de conversion de [Su $(O\text{-}A)_{n-1}$-Y], noté [Su P-Y] supérieur à 95 %,
- d'élimination du solvant aprotique polaire,
- de reprise du résidu par un solvant organique,
- d'élimination du sel de l'acide dithiocarbamique résiduel,
- d'évaporation de la phase organique contenant l'ester dithiocarbamique,
- de purification de cet ester.

8. Procédé selon la revendication 7, caactérisé en ce que le sel de l'acide dithiocarbamique est un sel de métal alcalin.

9. Procédé selon la revendication 8, caractérisé en ce que le sel du métal alcalin de l'acide dithiocarbamique est le sel de lithium, de sodium ou de potassium.

10. Procédé selon l'une quelconque des revendications 7 à 9, caractérisé en ce que le sel de l'acide dithiocarbamique est ajouté au solvant aprotique polaire dans un rapport molaire généralement compris entre 1,1 et 2, et de préférence voisin de 1,5 par rapport a l'intermédiaire [Su $(O\text{-}A)_{n-1}$- Y], noté [Su P-Y] .

11. Procédé selon l'une quelconque des revendications 7 à 10, caractérisé en ce que le solvant aprotique polaire est choisi dans le groupe constitué par l'acétone, l'hexaméthyl phosphorotriamide, le diméthylformamide, le diméthoxyéthane, le diméthylsulfoxyde ou encore par un mélange de ces solvants.

12. Procédé selon l'une quelconque des revendications 7 à 11, caractérisé en ce que le solvant aprotique polaire ou le mélange de solvants aprotiques polaires est associé à un autre solvant organique.

13. Procédé selon la revendication 12, caractérisé en ce que le solvant organique associé au solvant aprotique polaire est un solvant aromatique tel que le toluène, le xylène, le benzène.

14. Procédé selon la revendication 12, caractérisé en ce que le solvant organique associé au solvant aprotique polaire est un hydrocarbure saturé ou encore un mélange d'hydrocarbures saturés.

15. Procédé selon la revendication 7, caractérisé en ce que le solvant organique dans lequel est repris le résidu, après élimination du solvant aprotique polaire, est l'éther éthylique.

16. Procédé selon la revendication 7, caractérisé en ce que l'on élimine, après reprise du résidu dans le solvant organique, le sel de l'acide dithiocarbamique résiduel, par filtration.

17. Procédé selon la revendication 7, caractérisé en ce que l'on purifie l'ester dithiocarbamique par recristallisation ou par chromatographie liquide sur gel de silice.

18. Procédé selon la revendication 7, caractérisé en ce que, dans le cas des esters sulfoniques, on procède à une filtration afin d'éliminer le sulfonate formé.

19. Procédé selon l'une quelconque des revendications 1 et 2, caractérisé en ce que le polyol Su (OH) est choisi parmi le glycérol, le glucose, le galactose, le mannose, le fructose, des itols ou des mono-, di- ou polysaccharides.

20. Procédé selon l'une quelconque des revendications précédentes, caractérisé en ce qu'il comprend la déprotection sélective ou totale des sites OH bloqués de l'ester dithiocarbamique par catalyse acide homogène dans un mélange eau/alcanol, alcanol pur ou mélange d'alcanols ou acidocatalyse en phase pseudohomogène ou en phase hétérogène sur résine.

21. Procédé selon la revendication 20, caractérisé en ce que la déprotection sélective ou totale des sites OH bloqués de l'ester dithiocarbamique s'effectue par passage d'une solution de l'ester dithiocarbamique dans un mélange de solvants eau/alcanol, alcanol pur ou mélange d'alcanols sur une colonne remplie de résine acide maintenue à une température contrôlée.

22. Esters de l'acide dithiocarbamique de formule

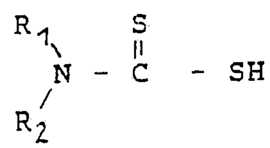

dans laquelle $R_1$ et $R_2$ sont des radicaux alkyles saturés ou non, substitués ou non avec des groupements carbonés ou des hétéroatomes, ou encore formant avec l'atome d'azote un cycle saturé ou non comportant ou non des hétéroatomes et du glycérol, sous forme acétal ou déprotégés, à l'exclusion du DEDTC de glycérol.

23. Esters de l'acide dithiocarbamique de formule

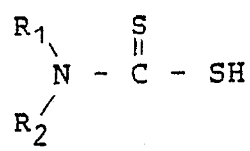

dans laquelle $R_1$ et $R_2$ ont la signification précitée et du galactose, sous forme acétal ou déprotégés, à l'exclusion du DMDTC de diacétone galactose.

24. Esters de l'acide dithiocarbamique de formule

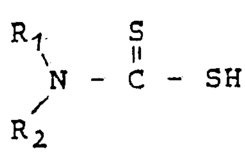

dans laquelle $R_1$ et $R_2$ ont la signification précitée et du glucose, sous forme acétal ou déprotégés.

25. Esters de l'acide dithiocarbamique de formule

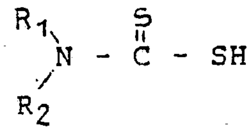

dans laquelle $R_1$ et $R_2$ ont la signification précitée et des itols, sous forme acétal ou déprotégés.

26. Le désoxy-1 N,N-diéthyl dithiocarbamate-1 O-isopropylidène-2,3 glycérol.

27. Le désoxy-1 N,N-diméthyl dithiocarbamate-1 glycèrol.

28. Les di-O-butanoyl (ou palmitoyl)-2,3 désoxy-1 N,N diéthyl (ou diméthyl) dithiocarbamate-1-glycérols.

29. Les désoxy-3 diéthyl (ou diméthyl) dithiocarbamate-3 di-O-isopropylidène 1,2:5,6 -D allofurannoses.

30. Le désoxy-1 N,N diéthyl dithiocarbamate-1 di-O-isopropylidène 2,3 : 4,5 -D fructopyrannose.

31. Le désoxy-6 N,N-diéthyl dithiocarbamate-6 di-O-isopropylidène-1,2 : 3,4 - - D galactopyrannose.

32. Les désoxy-3 N,N-diéthyl (ou diméthyl) dithiocarbamate-3 di-O-isopropylidène-1,2 : 5,6- - D glucofurannoses.

33. Les désoxy-3 N,N-diéthyl (ou diméthyl) dithiocarbamate-3 O-isopropylidène 1,2- - D glucofurannoses.

34. Les désoxy-3 N,N-diéthyl (ou diméthyl) dithiocarbamate-3 D-glucopyrannoses.

35. Médicament immunomodulateur caractérisé en ce qu'il contient, à titre de principe actif, l'un des esters dithiocarbamiques définis dans les revendications 22 à 34.

36. Médicament pour le traitement des maladies virales, notamment celles dues aux virus HIV 1 ou 2, caractérisé en ce qu il contient, à titre de principe actif, l'un des esters dithiocarbamiques définis dans les revendications 22 à 34.

37. Les dithiocarbamates de lithium, y compris le DMDTC-Li et le DEDTC-Li.

38. Médicament immunomodulateur caractérisé en ce qu'il contient, à titre de principe actif, un composé selon la revendication 37.

39. Médicament pour le traitement des maladies virales, notamment celles dues aux virus HIV 1 ou 2, caractérisé en ce qu'il contient, à titre de principe actif, un composé selon la revendication 37.